# EUROPEAN PATENT SPECIFICATION

(11) **EP 3 819 640 B1**
(45) Date of publication and mention of the grant of the patent: **18.06.2025**
(21) Application number: 19830028.7
(22) Date of filing: 05.07.2019
(51) Int. Cl.: G01N 33/86

(54) **LIPOSOME-CONTAINING LIQUID REAGENT FOR MEASURING BLOOD COAGULATION ABILITY**
LIPOSOMHALTIGES FLÜSSIGES REAGENZ ZUR MESSUNG DER BLUTGERINNUNGSFÄHIGKEIT
RÉACTIF LIQUIDE CONTENANT DES LIPOSOMES POUR MESURER LA CAPACITÉ DE COAGULATION SANGUINE

(30) Priority: 06.07.2018 JP 2018128794
(43) Date of publication of application: 12.05.2021
(73) Proprietor: PHC Corporation, Toon-shi Ehime 791-0395 (JP)
(72) Inventor: KADOWAKI, Atsushi, Ehime 791-0395 (JP)
(74) Representative: Gille Hrabal Partnerschaftsgesellschaft mbB Patentanwälte
(86) International application number: PCT/JP2019/026804
(87) International publication number: WO 2020/009221

(56) References cited:
- EP-A1- 3 637 107
- JP-A- 2003 510 610
- JP-A- 2003 516 525
- JP-A- 2004 191 320
- JP-A- 2004 528 534
- JP-A- 2008 530 208
- JP-A- 2017 181 265
- JP-A- S61 502 908
- US-A- 4 529 561
- M. OKUDA ET AL: "Usefulness of synthetic phospholipid in measurement of activated partial thromboplastin time: a new preparation procedure to reduce batch difference", CLINICAL AND LABORATORY HAEMATOLOGY, vol. 26, no. 3, 1 June 2004 (2004-06-01), OXFORD, pages 215 - 223, XP055667435, ISSN: 0141-9854, DOI: 10.1111/j.1365-2257.2004.00605.x
- DUDEK M. M. ET AL: "Evaluation of activated partial thromboplastin time (aPTT) reagents for application in biomedical diagnostic device development : APTT REAGENTS FOR APPLICATION IN BIODEVICES", INTERNATIONAL JOURNAL OF LABORATORY HEMATOLOGY, vol. 33, no. 3, 1 December 2010 (2010-12-01), GB; US, pages 272 - 280, XP055897602, ISSN: 1751-5521, DOI: 10.1111/j.1751-553X.2010.01283.x
- TSUDA TOMOHIDE ET AL: "Effect of phosphatidylcholine, phosphatidylethanolamine and lysophosphatidylcholine on the activated factor X-prothrombin system.", BLOOD COAGULATION & FIBRINOLYSIS : AN INTERNATIONAL JOURNAL IN HAEMOSTASIS AND THROMBOSIS SEP 2006, vol. 17, no. 6, September 2006 (2006-09-01), pages 465 - 469, XP009533975, ISSN: 0957-5235

## Description

### TECHNICAL FIELD

The present invention relates to a liposome-containing liquid reagent for measuring blood coagulation ability.

### BACKGROUND ART

Among clinical tests, the blood coagulation ability test, which examines the pathological condition caused by the activity of blood coagulation factors, is a commonly performed test. Among them, an inspection method containing liposomes, which are bilayer membranes composed of phospholipids, is used. An activated partial thromboplastin time (APTT) measurement, a prothrombin time (PT) measurement, a coagulation factor quantitative test using the APTT measurement or the PT measurement, a complex factor measurement, a quantitative test for coagulation inhibitors, and the like may be exemplified.

Liposomes are bilayer membranes composed of phospholipids derived from a living body. Liposomes are applied to pharmaceutical formulations, cosmetic materials, in vitro diagnostic materials, drug delivery systems, or the like.

The blood coagulation reaction is achieved by a cascade of a plurality of coagulation factors, most of which proceeds on the membrane surface of phospholipids. Negative charges such as phosphatidylserine (PS) or phosphatidylglycerol (PG) are important for the hydrophilic group portion of phospholipids to bind to coagulation factors. On the other hand, it is known that the fluidity of the phospholipid membrane caused by the fatty acid side chain of the phospholipid also greatly affects the coagulation reaction. Liposomes containing unsaturated fatty acids that increase fluidity are known to be more active.

Physiological hemostasis includes an extrinsic system initiated by the binding of tissue factor (TF) exposed at the site of injury and activated factor VII, and an intrinsic coagulation system initiated independently of TF. Bleeding and pathological thrombus formation are thought to be due to the extrinsic system. APTT reproduces this reaction in vitro. In APTT, a reagent is added to test plasma, and the time required for thrombin produced by activation of each coagulation factor to convert fibrinogen to fibrin is measured. APTT detects intrinsic system (factors XII, XI, IX, VIII, X, V, and II, and fibrinogen) abnormalities. It is also used for a quantification of intrinsic coagulation factor activity, which is a detailed test for APTT abnormalities, a detection of acquired hemophilia and lupus anticoagulant, a cross-mixing test, which is a differential test, or monitoring of heparin administration.

The performance required for APTT is that it is highly sensitive to blood coagulation factors, and anticoagulants such as heparin. Since APTT is an item that needs to be diagnosed based on the absolute value of coagulation time, differences in production lots are more likely to appear compared to items for which a general calibration curve is created to obtain a diagnostic value. Therefore, in particular, a reagent with a small difference in production lots is a clinically highly preferred performance.

Various studies have been conducted on the difference in production lots of APTT. With respect to conventional APTT reagents, naturally occurring phospholipids such as soybeans were extracted and used to prepare APTT reagents. By converting phospholipids from natural sources to synthetic products, uniform phospholipids can be obtained, which has contributed to the reduction of the difference in production lots (Non-patent literature 1). However, the difference in production lots has not completely disappeared, and there is still room for improvement in order to meet clinical demands.

For example, Patent literature 1 describes that a liposome-containing liquid was obtained by filtering through a 0.6 µm polycarbonate membrane to uniform the particle size of liposome. Since this is an operation before reconstitution of tissue factor into synthetic liposomes, and it is an operation before freeze-drying, it is unlikely that the effect of making the particle size uniform by filtering a final product through the polycarbonate membrane is obtained. Furthermore, the effect shown in Patent literature 1 is a reduction of variation (difference) in measured prothrombin time before and after lyophilization, and the absolute value of coagulation time for each production lot is not controlled.

The documents M. Okuda et al., Clinical and Laboratory Haematology, vol. 26, no. 1, 2004, 215-223 and M.M. Dudek et al., International Journal of Laboratory Haematology, vol. 33, no. 3, 2010, 272-280 both disclose the preparation of several formulations of liposomes for use in APTT (activated patial thromboplastin time) for measuring the coagulation time in blood samples.

T. Tsuda et al., Blood Coagulation & Fibrinolysis, vol. 17, no. 6, 2006, 465-469 discloses a study examining the effetcs of phosphatidylcholine on the blood coagulation system using liposomes containing varying concentrations of phosphatidylcholine in the presence of phosphatidylserine at a constant concentration.

### CITATION LIST

### PATENT LITERATURE

[Patent literature 1] Japanese Unexamined Patent Publication (Kokai) No. 2017-181265

### NON-PATENT LITERATURES

[Non-patent literature 1] Okuda M, Kikukawa N, Uemura Y, "Development of new APTT reagent using synthetic phospholipid", Journal of the Japanese Society for Laboratory Hematology, February 28, 2002, vol. 3, no. 1, p. 124-131, reprint

### SUMMARY OF INVENTION

### TECHNICAL PROBLEM

The present invention has been made in view of these problems, and an object of the present invention is to provide a liposome-containing liquid reagent for measuring blood coagulation ability with a small difference in production lots.

### SOLUTION TO PROBLEM

The present inventor conducted intensive studies in view of the object to find a surprising result that, contrary to conventional expectations, the coefficient of variation between lots changed significantly depending on the particle size of liposomes. Based on this finding, the present invention has succeeded in producing a liposome-containing liquid reagent for measuring blood coagulation ability with a small difference in production lots by controlling the cumulative scattering intensity distributionof liposomes with particle sizes of 351 nm to the maximum particle size to 0% or more and less than 35% relative to the cumulative scattering intensity distributionof total liposomes.

The present invention relates to:
[1] a liposome-containing liquid reagent for measuring blood coagulation ability, wherein a cumulative scattering intensity distributionof liposomes with particle sizes of 351 nm to a maximum particle size is 0% or more and less than 35% relative to a cumulative scattering intensity distribution of total liposomes, wherein the cumulative scattering intensity distribution of liposomes with a particle size of 351 nm to a maximum particle size is a value obtained by a dynamic light scattering method,
[2] the reagent of [1], wherein the reagent is an activated partial thromboplastin time measuring reagent, a prothrombin time measuring reagent, a complex factor measuring reagent, a specific factor activity measuring reagent, or a blood coagulation factor inhibitor measuring reagent,
[3] a process of manufacturing a liposome-containing liquid reagent for measuring blood coagulation ability, said process comprising:
   (step A): the step of mixing phospholipids;
   (step B): the step of forming liposomes by dispersing the mixed phospholipids in an aqueous solution; and
   (step C): the step of preparing uniform liposomes from the liposomes using a liposome particle size adjusting means, wherein a cumulative scattering intensity distribution of liposomes with particle sizes of 351 nm to a maximum particle size is 0% or more and less than 35% relative to a cumulative scattering intensity distribution of total liposomes, and
[4] a method for reducing a difference in production lots of a liposome-containing liquid reagent for measuring blood coagulation ability, wherein a cumulative scattering intensity distribution of liposomes with particle sizes of 351 nm to a maximum particle size is 0% or more and less than 35% relative to a cumulative scattering intensity distribution of total liposomes, wherein the cumulative scattering intensity distribution of liposomes with a particle size of 351 nm to a maximum particle size is a value obtained by a dynamic light scattering method.

### ADVANTAGEOUS EFFECTS OF INVENTION

In a reagent for measuring blood coagulation ability in which phospholipids are contained and the phospholipids form liposomes, the present invention has a remarkable effect of enabling highly accurate blood coagulation measurement by reducing the difference in production lots.

### BRIEF DESCRIPTION OF THE DRAWINGS

[Fig. 1] Figure 1 is a graph showing the particle size distribution of Liposome 1 (Example 2) and Liposome 2 (Comparative Example 1).
[Fig. 2] Figure 2 is a graph showing the cumulative scattering intensity distribution of Liposome 1 (Example 2) shown in Figure 1.
[Fig. 3] Figure 3 is a graph showing the cumulative scattering intensity distribution of Liposome 2 (Comparative Example 1) shown in Figure 1.
[Fig. 4] Figure 4 is a graph showing the result of calculating the coefficient of variation (CV value) in each lot by performing ATPP measurement using APTT reagent <<Example 2>> and APTT reagent <<comparative Example 1>.
[Fig. 5] Figure 5 is a graph showing the particle size distribution of Liposome 3 (Example 5) and Liposome 4 (Comparative Example 2).
[Fig. 6] Figure 6 is a graph showing the cumulative scattering intensity distribution of Liposome 3 (Example 5) shown in Figure 5.
[Fig. 7] Figure 7 is a graph showing the cumulative scattering intensity distribution of Liposome 4 (Comparative Example 2) shown in Figure 5.
[Fig. 8] Figure 8 is a graph showing the result of calculating the coefficient of variation (CV value) in each lot by performing ATPP measurement using APTT reagent <<Example 5>> and APTT reagent <<comparative Example 2>.
[Fig. 9] Figure 9 is a graph showing the particle size distribution of Liposome 5 (Comparative Example 3).
[Fig. 10] Figure 10 is a graph showing the cumulative scattering intensity distribution of Liposome 5 (Comparative Example 3) shown in Figure 9.

### DESCRIPTION OF EMBODIMENTS

### (Liposomes)

Liposomes that can be used in the present invention may be prepared in accordance with known methods, except that the liposomes are prepared so that the cumulative scattering intensity distribution of liposomes with particle sizes of 351 nm to the maximum particle size is 0% or more and less than 35% relative to the cumulative scattering intensity distribution of the total liposomes.

As a process of preparing liposomes, it comprises, for example,
step A: the step of mixing phospholipids;
step B: the step of forming liposomes by dispersing the mixed phospholipid mixture in an aqueous solution; and
step C: the step of preparing uniform liposomes from the liposome-dispersed phospholipid mixture using a liposome particle size adjusting means.

As step A, the phospholipid mixture may be prepared, for example, by mixing phospholipids; preferably by mixing phospholipids followed by adding a fat-soluble antioxidant, or by mixing phospholipids in the presence of a fat-soluble antioxidant (fat-soluble-antioxidant-added phospholipid mixture). The phospholipid may be a naturally occurring phospholipid or a synthetic phospholipid. Those skilled in the art can appropriately select and use from known phospholipids, and can appropriately select and combine from phospholipids derived from plants or animals, as well as synthetic phospholipids selected from phosphatidylcholine, phosphatidylserine, phosphatidylethanolamine, phosphatidylinositol, phosphatidylglycerol, and the like. The fatty acid side chain of these phospholipids is preferably one containing an unsaturated fatty acid. The phospholipid mixture may be prepared in accordance with known methods.

There may be mentioned, for example, a method of forming a mixed phospholipid membrane by adding a fat-soluble antioxidant, such as butylhydroxytoluene or α-tocopherol, to phospholipid dissolved in an organic solvent, such as chloroform, and removing the organic solvent from the preparation.

As step B, there may be mentioned, for example, a method of forming liposomes by dispersing the phospholipid membrane in an aqueous solution. As the aqueous solution, a buffer (HEPES, TRIS, PBS, or the like) may be exemplified, and the aqueous solution may contain a polymer, such as a protein, as appropriate.

As step C, for example, a known liposome particle size adjusting means may be appropriately selected to prepare uniform liposomes that can be used in the present invention. As the liposome particle size adjusting means, any means may be used as long as the particle size of the uniform liposomes that can be used in the present invention can be obtained, and filtration through a filter, ultrasonication, or the like may be exemplified. The material, the membrane pore size, the strength, the time, the volume, or the like can be appropriately selected and implemented by those skilled in the art according to the target particle size.

As the filtration through a filter, a polycarbonate membrane, a cellulose acetate membrane, or the like may be used. As the ultrasonication, a probe-type ultrasonic generator, a bath-type ultrasonic generator, or the like may be used. The filtration is preferable because uniform liposomes can be prepared more easily.

With respect to the particle size of liposomes that can be used in the present invention, the cumulative scattering intensity distribution of liposomes with particle sizes of 351 nm to the maximum particle size is 0% or more and less than 35% relative to the cumulative scattering intensity distribution of the total liposomes, preferably 0% or more and less than 30%, more preferably 0% or more and less than 25%, still more preferably 0% or more and less than 20%, and most preferably 0% or more and less than 15%.

The minimum particle size is not limited as long as the liposomes function as a scaffold, but those skilled in the art can appropriately select the particle size to prepare the liposomes. Preferably, the cumulative scattering intensity distribution of liposomes with particle sizes of 53 nm to the minimum particle size is 0% or more and less than 54% relative to the cumulative scattering intensity distribution of the total liposomes, preferably 0% or more and less than 50%, more preferably 0% or more and less than 40%, still more preferably 0% or more and less than 30%, and most preferably 0% or more and less than 20%.

Furthermore, the maximum particle size is preferably 650 nm or less, more preferably 600 nm or less, still more preferably 50 nm to 600 nm, and still more preferably 100 nm to 400 nm. The minimum particle size is 0 nm or more, preferably 10 nm or more, more preferably 20 nm or more and 50nm or less, and still more preferably 30 nm or more and less than 66 nm.

As a process of manufacturing a reagent for measuring blood coagulation ability that can be performed in the present invention, the process may be performed in accordance with known processes of manufacturing a reagent for measuring blood coagulation ability, except that the liposomes are prepared so that the cumulative scattering intensity distribution of liposomes with particle sizes of 351 nm to the maximum particle size is 0% or more and less than 35% relative to the cumulative scattering intensity distribution of the total liposomes. As shown in the Examples described below, it is preferable that the cumulative scattering intensity distribution of liposomes with particle sizes of 351 nm to the maximum particle size is 0% or more and less than 35% relative to the cumulative scattering intensity distribution of the total liposomes, because the difference in production lots can be reduced.

In connection with this, the particle size of liposomes may be measured as the particle size of liposomes alone, or may be measured as the particle size of liposomes with other additives added. As the additives, an activator in APTT may be exemplified.

### (Tissue factor)

Tissue factor that can be used in the present invention can be appropriately selected and used by those skilled in the art from known tissue factors. Either natural tissue factor or recombinant tissue factor may be applied, but recombinant tissue factor is preferred. As the recombinant tissue factor, a recombinant protein produced by a host, such as an insect cell infected with a virus into which the gene sequence of human or animal tissue factor is integrated, recombinant Escherichia coli or yeast, can be used. The recombinant tissue factor can be prepared in accordance with known methods. For example, since tissue factor expressed in the host is a transmembrane protein and insoluble in a simple aqueous solvent, the tissue factor can be separated from the expressing cells by disruption extraction with a buffer containing a surfactant, and the separated tissue factor can be purified by an operation such as chromatography.

### (Lipidated thromboplastin)

Lipidated thromboplastin that can be used in the present invention may be composed of, for example, a tissue factor solution and a fat-soluble-antioxidant-added phospholipid mixture containing liposomes (fat-soluble-antioxidant-added lipidated thromboplastin). Although the lipidated thromboplastin can be appropriately selected and used by those skilled in the art from known methods for producing lipidated thromboplastin, for example, a tissue factor solution and a fat-soluble-antioxidant-added phospholipid mixture containing liposomes may be mixed at a molar ratio of approximately 1:1000 to 1:500000 to obtain a uniform solution. The tissue factor solution is a buffer containing a surfactant sufficient to dissolve tissue factor and a fat-soluble-antioxidant-added phospholipid mixture containing liposomes, and may further contain serum albumin, globulin, or the like as a protective protein. After sufficiently mixing and dissolving the tissue factor solution and the fat-soluble-antioxidant-added phospholipid mixture containing liposomes, liposomes composed of phospholipids are formed by removing the surfactant, and at the same time, the transmembrane portion of the tissue factor is incorporated into the phospholipid membrane, and as a result, a fat-soluble-antioxidant-added lipidated thromboplastin is prepared. As the method for removing the surfactant, a known dialysis method or a method using an adsorption resin can be applied.

### (Liquid reagent and method for measuring blood coagulation ability)

The liquid reagent for measuring blood coagulation ability that can be used in the present invention is not limited as long as it is produced as a liquid reagent using a liposome-containing reagent for measuring blood coagulation ability, and for example, an activated partial thromboplastin time measuring reagent, a prothrombin time measuring reagent, a complex factor measuring reagent, a specific factor activity measuring reagent, or a blood coagulation factor inhibitor measuring reagent may be exemplified. Those skilled in the art can appropriately produce and use the reagent using known information.

The embodiment of the liquid reagent and method for measuring blood coagulation ability of the present invention may be a one-component reagent, or a formulation consisting of a plurality of reagents, such as a two-component reagent. More particularly, when it is provided as an activated partial thromboplastin time measuring reagent, not only can it be provided as a one-reagent-based formulation in which liposomes, which are the main reaction component of the reagent, an activator typified by colloidal silica or ellagic acid, and a calcium ion, which is coagulation factor IV, are mixed, but it can also be provided, as a formulation with longer-term storage stability, as a two-reagent-based formulation in which liposomes and an activator are separated from an aqueous solution of calcium compound.

When a PT measuring reagent is provided as a liquid reagent, for example, not only can it be provided as a one-reagent-based formulation in which lipidated thromboplastin (coagulation factor III), which is the main reaction component of the reagent, and a calcium ion, which is coagulation factor IV, are mixed, but it can also be provided, as a formulation with longer-term storage stability, as a two-reagent-based formulation in which lipidated thromboplastin is separated from an aqueous solution of calcium compound.

As the first embodiment of the liquid reagent and method for measuring blood coagulation ability of the present invention, it can be used as an activated partial thromboplastin time measuring reagent. More particularly, the activated partial thromboplastin time measuring reagent is provided by adding a calcium ion at a concentration capable of exhibiting blood coagulation activity to liposomes and an activator typified by colloidal silica or ellagic acid. A preservative, a pH buffer, an additive to avoid the effect of an anticoagulant that affects a PT measurement, or the like can be added to the reagent.

As the second embodiment of the liquid reagent and method for measuring blood coagulation ability of the present invention, it can be used as a PT measuring reagent. More particularly, the PT measuring reagent is provided by adding a calcium ion at a concentration capable of exhibiting blood coagulation activity to lipidated thromboplastin. A preservative, a pH buffer, an additive to avoid the effect of an anticoagulant that affects a PT measurement, or the like can be added to the reagent.

As the third embodiment of the liquid reagent and method for measuring blood coagulation ability of the present invention, it can be used as a complex factor measuring reagent prepared by mixing one configured in the same manner as the PT measurement reagent with plasma excluding factor V among coagulation factors (factor II, factor V, factor VII, and factor X)(deficient plasma: i.e., containing factor II, factor VII, and factor X as coagulation factors) to test the activities of factor II, factor VII, and factor X.

The third liquid reagent for measuring blood coagulation ability may be configured as a two-component reagent in which the PT measuring reagent and the deficient plasma are separately prepared, or may be configured as a one-component reagent that is prepared by mixing the PT measuring reagent and the deficient plasma. As an example of the two-component reagent, it is exemplified that when the first reagent is the PT measurement reagent, and the second reagent is the deficient plasma, a sample is reacted with the first reagent, and then the deficient plasma is reacted to perform the measurement. As an example of the one-component reagent, it is exemplified that the reagent in which the PT measurement reagent and the deficient plasma are mixed is configured, and is reacted with a sample to perform the measurement. Those skilled in the art can appropriately select which reagent configuration should be adopted, and perform it.

As the fourth embodiment of the liquid reagent and method for measuring blood coagulation ability of the present invention, it can be used as a specific factor activity measuring reagent prepared by combining one configured in the same manner as the PT measurement reagent with factor-deficient plasma deficient in any one of coagulation factors (Factor II, Factor V, Factor VII, and Factor X) to test a specific factor activity. For example, deficient plasma of factor VII (i.e., containing factor II, factor V, and factor X as coagulation factors) can be combined with the PT measurement reagent to measure the activity of factor VII in a sample. Deficient plasma of factor II (i.e., containing factor V, factor VII, and factor X as coagulation factors) can be combined with the PT measurement reagent to measure the activity of factor II in a sample. Deficient plasma of factor X (i.e., containing factor II, factor V, and factor VII as coagulation factors) can be combined with the PT measurement reagent to measure the activity of factor X in a sample.

The fourth liquid reagent for measuring blood coagulation ability may be configured as a two-component reagent in which the PT measuring reagent and the deficient plasma are separately prepared, or may be configured as a one-component reagent that is prepared by mixing the PT measuring reagent and the deficient plasma. As an example of the two-component reagent, it is exemplified that when the first reagent is the deficient plasma, and the second reagent is the PT measurement reagent, a sample is reacted with the first reagent, and then the second reagent is reacted to perform the measurement. As an example of the one-component reagent, it is exemplified that the reagent in which the PT measurement reagent and the deficient plasma are mixed is configured, and is reacted with a sample to perform the measurement. Those skilled in the art can appropriately select which reagent configuration should be adopted, and perform it.

As the fifth embodiment of the liquid reagent and method for measuring blood coagulation ability of the present invention, it can be performed in the same manner as the third embodiment, except that the target sample is different. It is exemplified that it is configured in the same manner as the PT measurement reagent, and used as a blood coagulation factor inhibitor measuring reagent to test for the presence of a blood coagulation factor inhibitor in plasma.

The fifth liquid reagent for measuring blood coagulation ability may be configured as a two-component reagent in which the PT measuring reagent and the deficient plasma are separately prepared, or may be configured as a one-component reagent that is prepared by mixing the PT measuring reagent and the deficient plasma. As an example of the two-component reagent, it is exemplified that when the first reagent is the PT measurement reagent, and the second reagent is the deficient plasma, a sample is reacted with the first reagent, and then the deficient plasma is reacted to perform the measurement. As an example of the one-component reagent, it is exemplified that the reagent in which the PT measurement reagent and the deficient plasma are mixed is configured, and is reacted with a sample to perform the measurement. Those skilled in the art can appropriately select which reagent configuration should be adopted, and perform it.

The activity of each of the above-mentioned factors can be tested by evaluating the time until fibrin formation (coagulation) is observed (coagulation time) or by evaluating the time (%) relative to standard plasma. Those skilled in the art can appropriately select and perform the evaluation method according to the purpose.

### (Samples)

Examples of the sample that can be used in the present invention include blood, plasma obtained from blood, and the like according to known information. For example, plasma obtained from the blood of a subject, control plasma, a mixture thereof, or the like may be used. As the control plasma, normal plasma, plasma for quality control, or the like may be used. The normal plasma may be plasma obtained from the blood of a healthy person, or may be commercially available normal plasma.

### EXAMPLES

The present invention will now be further illustrated by, but is by no means limited to, the following Examples.

### <<Example 1: Preparation of phospholipid mixture (1)>>

Dioleylphosphatidylcholine, dioleylphosphatidylethanolamine, and dioleylphosphatidylserine (all purchased from NOF Corporation) were mixed in a weight ratio of 4:3:3, and were dissolved in chloroform to prepare a phospholipid solution. Next, a fat-soluble antioxidant butylhydroxytoluene (BHT: purchased from WAKO) was added so that the weight ratio to the phospholipids was 0.05%, and the solvent was removed from the solution by an evaporator and the residue was dried to prepare a phospholipid mixture with a fat-soluble antioxidant.

### <<Example 2: Preparation of Liposome 1>>

A buffer (10 mmol/L HEPES, 20 mmol/L sodium chloride, pH 7.5) was added to the phospholipid mixture obtained in Example 1, and stirred to disperse the phospholipid mixture in the buffer.

The dispersion was passed through a polycarbonate membrane with a pore size of 200 nm 10 times to make the particle size uniform. This solution was diluted 50-fold with a buffer (10 mmol/L HEPES, 20 mmol/L sodium chloride, 1% polyethylene glycol, pH 7.5) to prepare Liposome 1. The same operation was performed 3 times to obtain 3 lots.

### <<Comparative Example 1: Preparation of Liposome 2>>

A buffer (10 mmol/L HEPES, 20 mmol/L sodium chloride, pH 7.5) was added to the phospholipid mixture obtained in Example 1, and stirred to disperse the phospholipid mixture in the buffer.

The dispersion was diluted 50-fold with a buffer (10 mmol/L HEPES, 20 mmol/L sodium chloride, 1% polyethylene glycol, pH 7.5) to prepare Liposome 2. The same operation was performed 3 times to obtain 3 lots.

### <<Example 3: Examination of usefulness of liposomes (1)>>

### <<Particle size distribution measurement (1)>>

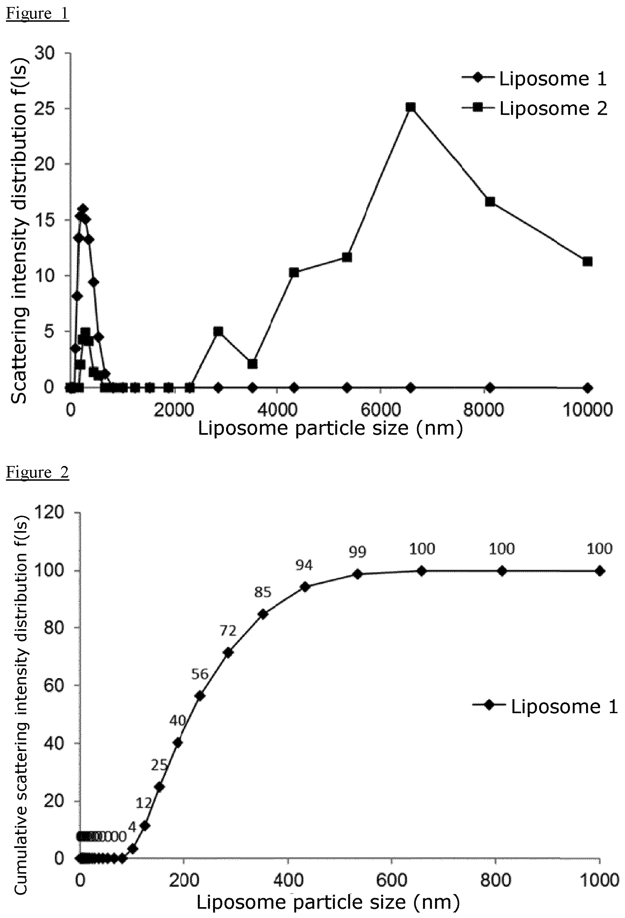

The particle size distribution of Liposome 1 and Liposome 2 respectively obtained in Example 2 and Comparative Example 1 was measured using a dynamic light scattering method (Otsuka Electronics PHOTAL ELSZ). The results are shown in Figure 1. It was found that Liposome 1 had a peak at 187 nm and was composed of liposomes of 81 nm or more and 658 nm or less, whereas Liposome 2 had a peak at 6580 nm and was composed of liposomes of 152 nm or more and up to at least 10000 nm.

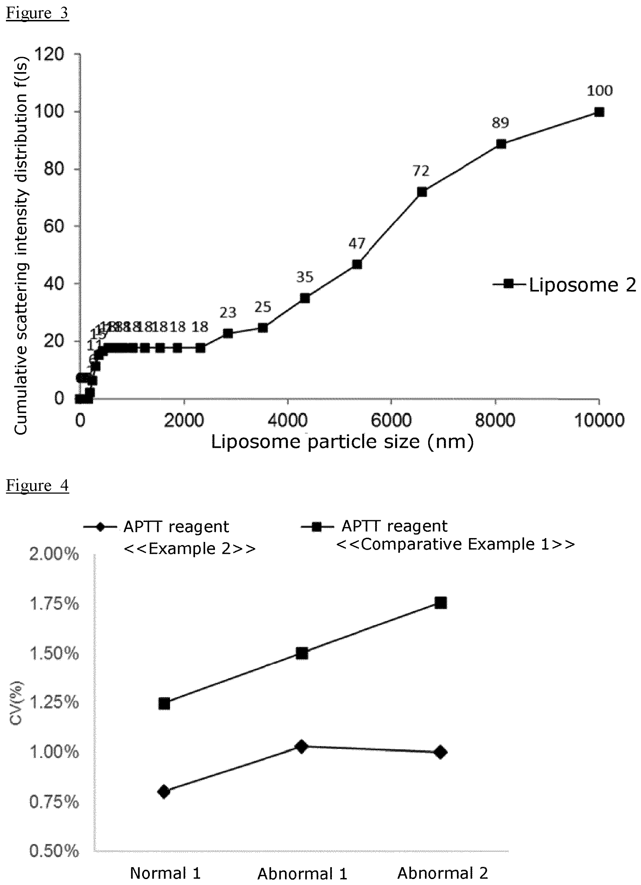

Furthermore, the cumulative scattering intensity distribution of Liposome 1 is shown in Figure 2, and the cumulative scattering intensity distribution of Liposome 2 is shown in Figure 3.

### <<Preparation of APTT reagent (1)>>

Liposome 1 and Liposome 2 were prescribed so that the phospholipid concentration was 100 mg/mL and the colloidal silica concentration was 0.05%, to prepare an APTT reagent <<Example 2>> and an APTT reagent <<comparative Example 1>>, respectively.

### <<Coagulation time measurement (1)>>

The measurement was performed using the APTT reagent <<Example 2>> and the APTT reagent <<comparative Example 1>>. A total of three types, i.e., Ci-Trol level 1: normal range 1, as normal control plasma; and Ci-Trol level 2: abnormal range 1 and Ci-Trol level 3: abnormal range 2, as abnormal control plasma (all purchased from SYSMEX), were used.

The measurement was performed using an automated clinical testing system STACIA (LSI Medience Corporation). Measurement parameters were as follows:
Sample: 50 µL
APTT reagent: 50 µL
20 mmol/L Calcium chloride aqueous solution: 50 µL
Measurement wavelength: 660 nm
The measurement was performed with a multiplicity of 5, and the average value was adopted as APTT, and the coefficient of variation (CV value) for each lot was calculated.

The results are shown in Figure 4. It was found that the APTT reagent <<Example 2>> had a smaller coefficient of variation between the three lots in both the normal range and the abnormal range in comparison with the APTT reagent <<Comparative Example 1>>. From this result, it can be said that there is a large difference in production lots for the APTT reagent comprising liposomes with a large particle size.

In connection with this, the cumulative scattering intensity distribution of Liposome 2 with particle sizes of 351 nm to the maximum particle size was 85% relative to the cumulative scattering intensity distribution of the total liposomes, and the cumulative scattering intensity distribution of Liposome 1 with particle sizes of 351 nm to the maximum particle size was 15% relative to the cumulative scattering intensity distribution of the total liposomes. Therefore, with respect to the particle size of liposomes, it was found that it is preferable that the cumulative scattering intensity distribution of liposomes with particle sizes of 351 nm to the maximum particle size is 15% or more and less than 85% relative to the cumulative scattering intensity distribution of the total liposomes.

### <<Example 4: Preparation of phospholipid mixture (2)>>

Dioleylphosphatidylcholine, dioleylphosphatidylethanolamine, and dioleylphosphatidylserine (all purchased from NOF Corporation) were mixed in a weight ratio of 6:2:2, and were dissolved in chloroform to prepare a phospholipid solution. Next, a fat-soluble antioxidant butylhydroxytoluene (BHT: purchased from WAKO) was added so that the weight ratio to the phospholipids was 0.05%, and the solvent was removed from the solution by an evaporator and the residue was dried to prepare fat-soluble-antioxidant-added phospholipid mixture 2.

### <<Example 5: Preparation of Liposome 3>>

A buffer (10 mmol/L HEPES, 20 mmol/L sodium chloride, pH 7.5) was added to the phospholipid mixture 2 obtained in Example 4, and stirred to disperse the phospholipid mixture in the buffer.

The dispersion was passed through a polycarbonate membrane with a pore size of 200 nm 10 times to make the particle size uniform. This solution was diluted 50-fold with a buffer (10 mmol/L HEPES, 20 mmol/L sodium chloride, 1% polyethylene glycol, pH 7.5) to prepare Liposome 3. The same operation was performed 3 times to obtain 3 lots.

### <<Comparative Example 2: Preparation of Liposome 4>>

A buffer (10 mmol/L HEPES, 20 mmol/L sodium chloride, pH 7.5) was added to the phospholipid mixture 2 obtained in Example 4, and stirred to disperse the phospholipid mixture in the buffer.

The dispersion was passed through a cellulose acetate membrane with a pore size of 200 nm. This solution was diluted 50-fold with a buffer (10 mmol/L HEPES, 20 mmol/L sodium chloride, 1% polyethylene glycol, pH 7.5) to prepare Liposome 4. The same operation was performed 3 times to obtain 3 lots.

### <<Example 6: Examination of usefulness of liposomes (2)>>

### <<Particle size distribution measurement (2)>>

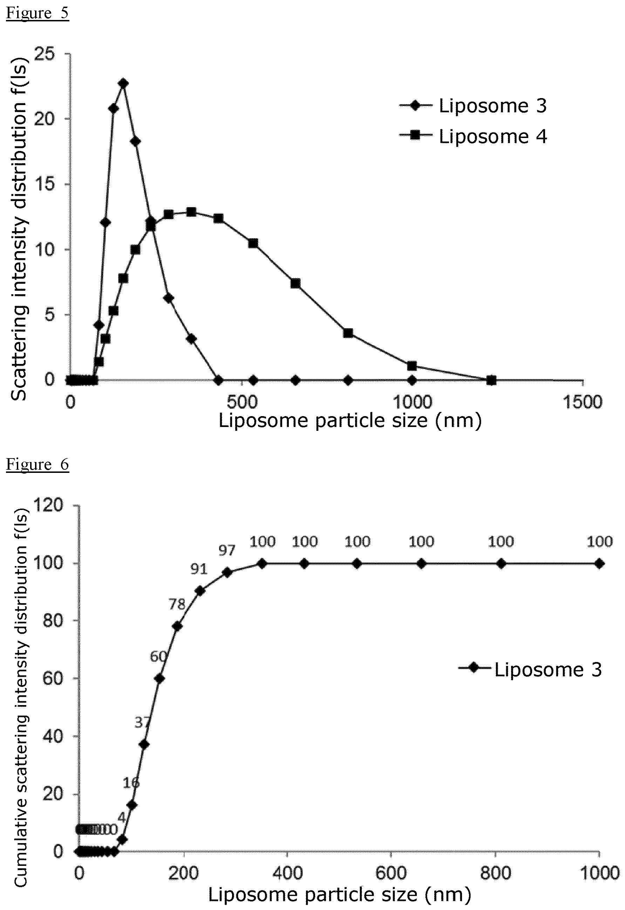

The particle size distribution of Liposome 3 and Liposome 4 was measured using a dynamic light scattering method (Otsuka Electronics PHOTAL ELSZ). The results are shown in Figure 5. Liposome 3 had a peak at 187 nm, whereas Liposome 4 had a peak at 351 nm.

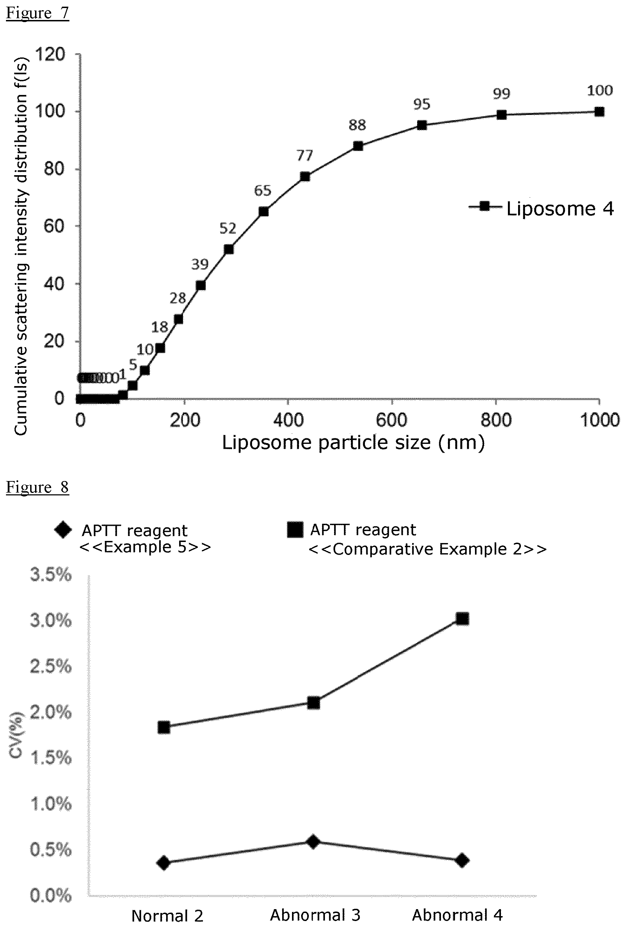

Furthermore, the cumulative scattering intensity distribution of Liposome 3 is shown in Figure 6, and the cumulative scattering intensity distribution of Liposome 4 is shown in Figure 7. In the cumulative scattering intensity distribution, total liposomes were 66 nm or more and 432 nm or less in Liposome 3, whereas total liposomes were 66 nm or more and 1232 nm or less in Liposome 4.

### <<Preparation of APTT reagent (2)>>

Liposome 3 and Liposome 4 were prescribed so that the phospholipid concentration was 100 mg/mL and the colloidal silica concentration was 0.05%, to prepare an APTT reagent <<Example 5>> and an APTT reagent <<comparative Example 2>>, respectively.

### << Coagulation time measurement (2)>>

The APTT measurement in normal range 2, abnormal range 3, and abnormal range 4 was performed using the APTT reagent <<Example 5>> and the APTT reagent <<comparative Example 2». An APTT measurement of a total of three types of control plasma, i.e., Normal Control (IL Company), as normal control plasma; and Low Abnormal Control and High Abnormal Control (IL Company), as abnormal control plasma, was performed.

The measurement was performed using an automated clinical testing system STACIA (LSI Medience Corporation). Measurement parameters were as follows:
Sample: 50 µL
APTT reagent: 50 µL
20 mmol/L Calcium chloride aqueous solution: 50 µL
Measurement wavelength: 660 nm
The measurement was performed with a multiplicity of 3, and the average value was adopted as APTT, and the coefficient of variation (CV value) for each lot was calculated.

The results are shown in Figure 8. It was found that the APTT reagent <<Example 5>> had a smaller coefficient of variation between the three lots in both the normal range and the abnormal range in comparison with the APTT reagent <<Comparative Example 2>>. From this result, it can be said that there is a large difference in production lots for the APTT reagent comprising liposomes with a large particle size.

In connection with this, the cumulative scattering intensity distribution of Liposome 4 with particle sizes of 351 nm (peak value) to the maximum particle size was 35% relative to the cumulative scattering intensity distribution of the total liposomes, and the cumulative scattering intensity distribution of Liposome 3 with particle sizes of 351 nm to the maximum particle size was 0% relative to the cumulative scattering intensity distribution of the total liposomes. Therefore, it was found that it is preferable that the cumulative scattering intensity distribution of liposomes with particle sizes of 351 nm to the maximum particle size is 0% or more and less than 35% relative to the cumulative scattering intensity distribution of the total liposomes.

Liposomes provide a reaction field for each coagulation factor, but are not involved in the reaction itself, and therefore, the inventor had considered that liposomes having a certain size would show a certain reactivity regardless of the size. However, it was a surprising result that the coefficient of variation between lots changed significantly depending on the size (particle size) of the liposomes.

### <<Comparative Example 3: Preparation of Liposome 5>>

A buffer (10 mmol/L HEPES, pH 7.5) was added to the phospholipid mixture obtained in Example 4, and stirred to disperse the phospholipid mixture in the buffer. The obtained solution was irradiated with ultrasonic waves by probe-type ultrasonic irradiation (BRANSON SONIFIER 450), and then, diluted 50-fold with buffer to obtain Liposome 5.

### <<Particle size distribution measurement (3)>>

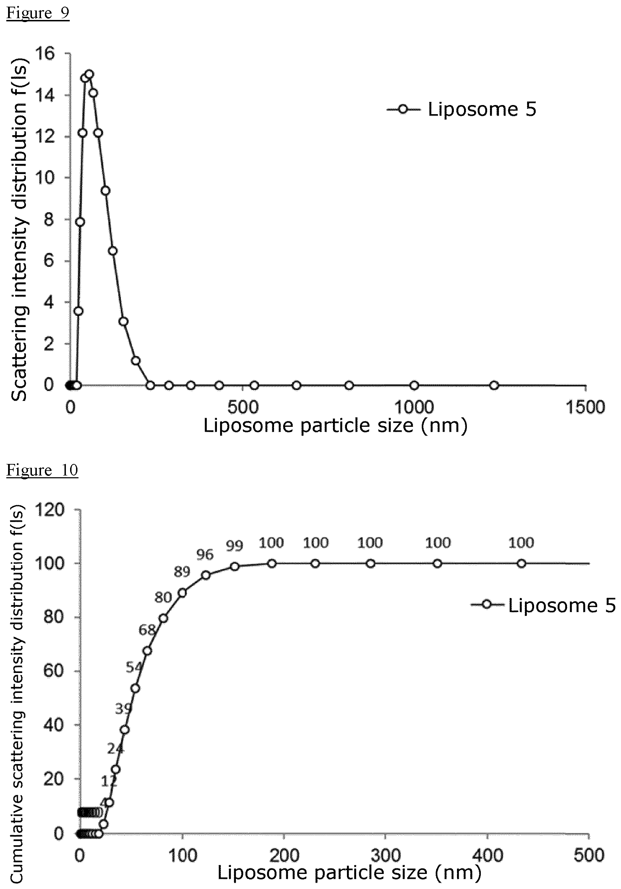

The particle size distribution of Liposome 5 was measured using a dynamic light scattering method (Otsuka Electronics PHOTAL ELSZ). The results are shown in Figure 9. It was found that Liposome 5 was composed of liposomes of 19 nm or more and 187 nm or less, and had a peak at 53 nm.

Furthermore, the cumulative scattering intensity distribution of Liposome 5 is shown in Figure 10. Of all liposomes, 96% was 123 nm or less.

### <<Preparation of APTT reagent (3)>>

Liposome 5 was prescribed so that the phospholipid concentration was 100 mg/mL and the colloidal silica concentration was 0.05%, to prepare an APTT reagent <<comparative Example 3».

### <<Coagulation time measurement (3)>>

The APTT measurement in normal range 2, abnormal range 3, and abnormal range 4 was performed using the APTT reagent <<Comparative Example 3>>. An APTT measurement of a total of three types of control plasma, i.e., Normal Control (IL Company), as normal control plasma; and Low Abnormal Control and High Abnormal Control (IL Company), as abnormal control plasma, was performed.

The measurement was performed using an automated clinical testing system STACIA (LSI Medience Corporation). Measurement parameters were as follows:
Sample: 50 µL
APTT reagent: 50 µL
20 mmol/L Calcium chloride aqueous solution: 50 µL
Measurement wavelength: 660 nm
The measurement was performed with a multiplicity of 3, and the average value was adopted as APTT, and the measurement was performed using an automated clinical testing system STACIA (LSI Medience Corporation).

Each coagulation time is shown in Table 1. In general, APTT reagents show a coagulation time of 25 to 35 seconds in the normal range, but it was found that, in Comparative Example 3, the normal control was 40 seconds or more, which exceeded the normal range of APTT reagents. From the results, it was found that liposomes that can be used as an APTT reagent is not desirable to be too small. Furthermore, it can be said that the minimum peak value of liposome particle size is 54 nm or more.

In connection with this, the cumulative scattering intensity distribution of Liposome 5 with particle sizes of 53 nm (peak value) to the maximum particle size was 54% relative to the cumulative scattering intensity distribution of the total liposomes. Therefore, it was found that it is preferable that the cumulative scattering intensity distribution of liposomes with particle sizes of 53 nm to the maximum particle size is 0% or more and less than 54% relative to the cumulative scattering intensity distribution of the total liposomes.

As the reason why it is not preferable to be too small, the following factors can be considered. For example, due to the small particle size of the liposomes, the liposomes cannot provide a sufficient place for the reaction field of each coagulation factor, so that the coagulation time is prolonged. Alternatively, since the measurements are made at the same lipid concentration, the number of liposome particles increases when the liposome particle size is small. Therefore, the probability that coagulation factors accumulate in one liposome decreases, and the coagulation time is prolonged.

**[Table 1]**

| | Normal 2 | Abnormal 3 | Abnormal 4 |
|---|---|---|---|
| Comparative Example 3 | 42.7 | 68.5 | 123.3 |

| | | | |
|---|---|---|---|
| sec. | | | |

## Claims

1. A liposome-containing liquid reagent for measuring blood coagulation ability, wherein a cumulative scattering intensity distribution of liposomes with a particle size of 351 nm to a maximum particle size is 0% or more and less than 35% relative to a cumulative scattering intensity distribution of total liposomes; and
wherein the cumulative scattering intensity distribution of liposomes with a particle size of 351 nm to a maximum particle size is a value obtained by a dynamic light scattering method.

2. The reagent according to claim 1, wherein the reagent is an activated partial thromboplastin time measuring reagent, a prothrombin time measuring reagent, a complex factor measuring reagent, a specific factor activity measuring reagent, or a blood coagulation factor inhibitor measuring reagent.

3. A process of manufacturing a liposome-containing liquid reagent for measuring blood coagulation ability, said process comprising:
step A: the step of mixing phospholipids;
step B: the step of forming liposomes by dispersing the mixed phospholipids in an aqueous solution; and
step C: the step of preparing uniform liposomes from the liposomes using a liposome particle size adjusting means, wherein a cumulative scattering intensity distribution of liposomes with a particle size of 351 nm to a maximum particle size is 0% or more and less than 35% relative to a cumulative scattering intensity distribution of total liposomes; and
wherein the cumulative scattering intensity distribution of liposomes with a particle size of 351 nm to a maximum particle size is a value obtained by a dynamic light scattering method.

4. A method for reducing a difference in production lots of a liposome-containing liquid reagent for measuring blood coagulation ability, wherein a cumulative scattering intensity distribution of liposomes with a particle size of 351 nm to a maximum particle size is 0% or more and less than 35% relative to a cumulative scattering intensity distribution of total liposomes, and
wherein the cumulative scattering intensity distribution of liposomes with a particle size of 351 nm to a maximum particle size is a value obtained by a dynamic light scattering method.

## Patentansprüche

1. Liposomenhaltiges Flüssigreagenz zur Messung der Blutgerinnungsfähigkeit, wobei eine kumulative Streuintensitätsverteilung von Liposomen mit einer Teilchengröße von 351 nm bis zu einer maximalen Teilchengröße 0 % oder mehr und weniger als 35 % relativ zu einer kumulativen Streuintensitätsverteilung der gesamten Liposomen beträgt; und
wobei die kumulative Streuintensitätsverteilung von Liposomen mit einer Partikelgröße von 351 nm bis zu einer maximalen Partikelgröße ein Wert ist, der durch eine dynamische Lichtstreuungsmethode erhalten wird.

2. Reagenz nach Anspruch 1, wobei es sich bei dem Reagenz um ein Reagenz zur Messung der aktivierten partiellen Thromboplastinzeit, ein Reagenz zur Messung der Prothrombinzeit, ein Reagenz zur Messung des Komplexfaktors, ein Reagenz zur Messung der spezifischen Faktoraktivität oder ein Reagenz zur Messung des Blutgerinnungsfaktorinhibitors handelt.

3. Verfahren zur Herstellung eines liposomenhaltigen Flüssigreagenzes zur Messung der Blutgerinnungsfähigkeit, wobei das Verfahren umfasst:
Schritt A: der Schritt des Mischens von Phospholipiden;
Schritt B: der Schritt der Bildung von Liposomen durch Dispergieren der gemischten Phospholipide in einer wässrigen Lösung; und
Schritt C: der Schritt der Herstellung einheitlicher Liposomen aus den Liposomen unter Verwendung eines Mittels zur Einstellung der Liposomenpartikelgröße, wobei eine kumulative Streuintensitätsverteilung der Liposomen mit einer Partikelgröße von 351 nm bis zu einer maximalen Partikelgröße 0 % oder mehr und weniger als 35 % relativ zu einer kumulativen Streuintensitätsverteilung der gesamten Liposomen beträgt; und
wobei die kumulative Streuintensitätsverteilung von Liposomen mit einer Partikelgröße von 351 nm bis zu einer maximalen Partikelgröße ein durch ein dynamisches Lichtstreuverfahren erhaltener Wert ist.

4. Verfahren zur Verringerung eines Unterschieds in den Produktionschargen eines liposomenhaltigen Flüssigreagenzes zur Messung der Blutgerinnungsfähigkeit, wobei eine kumulative Streuintensitätsverteilung von Liposomen mit einer Teilchengröße von 351 nm bis zu einer maximalen Teilchengröße 0 % oder mehr und weniger als 35 % relativ zu einer kumulativen Streuintensitätsverteilung der gesamten Liposomen beträgt, und
wobei die kumulative Streuintensitätsverteilung von Liposomen mit einer Partikelgröße von 351 nm bis zu einer maximalen Partikelgröße ein durch ein dynamisches Lichtstreuverfahren erhaltener Wert ist.

## Revendications

1. Réactif liquide contenant des liposomes pour mesurer la capacité de coagulation du sang, dans lequel la distribution cumulative de l'intensité de diffusion des liposomes dont la taille des particules est comprise entre 351 nm et la taille maximale des particules est égale ou supérieure à 0 % et inférieure à 35 % par rapport à la distribution cumulative de l'intensité de diffusion de l'ensemble des liposomes; et
dans lequel la distribution cumulative de l'intensité de diffusion des liposomes ayant une taille de particule de 351 nm à une taille de particule maximale est une valeur obtenue par une méthode de diffusion dynamique de la lumière.

2. Réactif selon la revendication 1, dans lequel le réactif est un réactif de mesure du temps de thromboplastine partielle activée, un réactif de mesure du temps de prothrombine, un réactif de mesure du facteur complexe, un réactif de mesure de l'activité du facteur spécifique, ou un réactif de mesure de l'inhibiteur du facteur de coagulation du sang.

3. Procédé de fabrication d'un réactif liquide contenant des liposomes pour mesurer la capacité de coagulation du sang, comprenant :
étape A: l'étape du mélange de phospholipides;
étape B: la formation de liposomes par dispersion des phospholipides mélangés dans une solution aqueuse; et
étape C: l'étape consistant à préparer des liposomes uniformes à partir des liposomes en utilisant un moyen d'ajustement de la taille des particules des liposomes, dans laquelle une distribution cumulative de l'intensité de diffusion des liposomes ayant une taille de particule comprise entre 351 nm et une taille de particule maximale est égale ou supérieure à 0 % et inférieure à 35 % par rapport à une distribution cumulative de l'intensité de diffusion de l'ensemble des liposomes; et
dans lequel la distribution cumulative de l'intensité de diffusion des liposomes dont la taille des particules est comprise entre 351 nm et une taille maximale est une valeur obtenue par une méthode de diffusion dynamique de la lumière.

4. Méthode pour réduire une différence entre les lots de production d'un réactif liquide contenant des liposomes pour mesurer la capacité de coagulation du sang, dans laquelle une distribution cumulative d'intensité de diffusion des liposomes avec une taille de particule de 351 nm à une taille de particule maximale est égale ou supérieure à 0 % et inférieure à 35 % par rapport à une distribution cumulative d'intensité de diffusion de l'ensemble des liposomes, et
dans lequel la distribution cumulative de l'intensité de diffusion des liposomes dont la taille des particules est comprise entre 351 nm et une taille maximale est une valeur obtenue par une méthode de diffusion dynamique de la lumière.
